Europäisches Patentamt

European Patent Office

Office européen des brevets

(19)

(11) Publication number: **0 341 519**
**A2**

(12) **EUROPEAN PATENT APPLICATION**

(21) Application number: **89107804.0**

(22) Date of filing: **28.04.89**

(51) Int. Cl.4: **G02C 7/10 , G02F 1/13**

(30) Priority: **11.05.88 JP 115209/88**
**20.05.88 JP 67181/88 U**
**23.05.88 JP 125171/88**
**23.05.88 JP 67754/88 U**

(43) Date of publication of application:
**15.11.89 Bulletin 89/46**

(84) Designated Contracting States:
**DE FR GB**

(71) Applicant: **SEIKO EPSON CORPORATION**
**4-1, Nishishinjuku 2-chome**
**Shinjuku-ku Tokyo-to(JP)**

(72) Inventor: **Okaue, Etsuo**
**c/o Seiko Epson Corporation 3-5, Owa**
**3-chome**
**Suwa-shi Nagano-ken(JP)**
Inventor: **Egawa, Masaru**
**c/o Seiko Epson Corporation 3-5, Owa**
**3-chome**
**Suwa-shi Nagano-ken(JP)**

(74) Representative: **Blumbach Weser Bergen**
**Kramer Zwirner Hoffmann Patentanwälte**
**Radeckestrasse 43**
**D-8000 München 60(DE)**

(54) **Electronic sunglasses.**

(57) Herein disclosed are a pair of electronic sunglasses (1) making use of the electrooptic effect of a liquid crystal. The sunglasses comprise a pair of liquid crystal panels of twist nematic type each made of a substrate of a synthetic resin for forming a transmittance-variable portion. The liquid crystal panels have a twist angle of an orientation of $60°$ to $80°$ or $100°$ to $120°$. Further comprised are a pair of polarizing plates applied to the liquid crystal panels and having their axes of absorption or polarization aligned with the angle of orientation of said liquid crystal panels. The smaller one of the angles formed between the axes of polarization of the objective polarizing plates of the liquid crystal panels for the right and left eyes is less than $45°$. A solar cell (2) acts as a power source for driving the liquid crystal panels.

FIG. 4

## Electronic Sunglasses

## BACKGROUND OF THE INVENTION

### Field of the Invention

The present invention relates to electronic sunglasses for controlling optical transmittance by making use of the electrooptic effect of a liquid crystal substance.

### Description of the Prior Art

In transmittance-variable sunglasses used widely in the prior art, the property of a photochromic substance to be colored on exposure to a light are utilized to either disperse the photochromic substance in the lenses or form photochromic, thin films on the surfaces of the lenses.

However, the sunglasses using such photochromic substance are defective: in that it takes a long time for the sunglasses to be colored on exposure to the light so that the coloring cannot be effected, as expected, in time when the sunglasses are exposed at night to the headlights of a car running in the oppo site direction; and in that the photochromic substance will age with the lapse of time so that the color becomes reluctant to come out even in a dark place.

A number of proposals for eliminating the defects of the photochromic sunglasses to provide sunglasses of quick response have been made by making use of the electrooptic effect of a liquid crystal substance. These electronic sunglasses employ the principle of the liquid crystal display, which is highlighted as one of low power consumption and thin size and is widely used at present, as will be described in the following.

Two substrates having their inner surfaces formed with transparent electrodes are filled up therein with a nematic liquid crystal and are so oriented that the molecules of the liquid crystal may intersect orthogonally at the portions contacting with one and the other substrates. As a result, the intermediate liquid crystal molecules twist spirally to polarize an incident light rotatorily by 90 degrees. The two substrates will transmit the incident light if they are equipped on their outer sides with polarizing plates such that their axes of polarization are oriented in parallel (i.e., with the two polarizing plates having orthogonal axes of polarization) with the directions of orientation of the substrates contacted by the polar izing plates. If, on the other hand, the liquid crystal molecules are supplied with a voltage, they are oriented in parallel with the direction of the voltage (i.e., normal to the two substrates), the spiral state is broken to allow the incident light to transmit through the liquid crystal molecules without being rotatorily polarized. Since, however, the two polarizing plates have the orthogonal axes of polarization, the incident light having transmitted through the liquid crystal molecules is screened by the polarizing plate at the emanating side.

If, on the contrary, the two polarizing plates are arranged to have their axes of polarization oriented in parallel, the incident light having been subjected to the rotatory polarization of 90 degrees is screened in the state of no voltage application because it orthogonally intersects the axis of polarization of the deflecting plate at the emanating side but can be allowed to transmit in the voltage-applied state because it is not rotatorily polarized.

According to either of the systems described above, it can be controlled depending upon the voltage application whether the incident light is allowed to transmit or screened.

The principle thus far described is called the "twist nematic type", on which are based most of the liquid crystal displays used widely at present. This principle is also applied to most of the electronic sunglasses proposed in the prior art. In the electronic sunglasses, more specifically, the liquid crystal panels of the twist nematic type are fitted in place of the lenses of the prior art to detect the brightness of the surroundings so that whether or not the voltage is to be applied to the liquid crystal panels may be accordingly determined to control the transmittance of the light. There are also proposed electronic sunglasses which use a solar cell as the voltage source so that the liquid crystal panels may be supplied with the voltage only when the surroundings are bright, i.e., when the solar cell generates its electromotive force.

Although a number of electronic sunglasses using the liquid crystal panels have been proposed heretofore, they are not put into practice yet because they are accompanied by the following defects.

Specifically, the substrates of the liquid crystal panels of the prior art are generally made of glass. This is partly because the strength has to be increased and partly because the gap between the two substrates to be filled up with a liquid crystal in the case of the liquid crystal display, i.e., the so-called "cell gap" has to be uniformalized so as to make the display contrast uniform.

However, the liquid panels using the glass substrates are too heavy for the electronic sunglasses, and an electric circuit and a power source are necessary for driving the liquid crystal panels so that the glasses are so heavy as a whole that they are defective in deteriorating the wearing feel so much that they cannot be worn continuously for a long time. Another serious safety problem is that the glass substrates are so fragile that they are broken by impacts to damage the wearer's eye balls or face fatally.

In the twist nematic type, on the other hand, the incident light is screened substantially completely if it is in the optically screening state. This screening is more desirable in the case of the display because the display contrast is improved. If, however, the field of vision is dropped to zero in the case of the sunglasses, a serious accident may possibly be invited during the drive of a car to raise a fatal problem.

Moreover, the twist nematic type liquid panels are well known to have the dependency upon the angle of vision, i.e., the property of varying the optical transmittance depending upon the angle of vision. In the case of the display, a serious problem will not occur if the display is placed at a considerable distance and at a substantially constant angle of vision. In the case of the electronic sunglasses, however, the liquid crystal panels are worn very near to the eye balls, and these eye balls are turned to look around so that the angle between the glance and the liquid crystal panels are highly varied. As a result, the transmittance is varied by the angle of vision so that the wearer feels a change in the shade or an unevenness to deteriorate the wearing feel, thus raising a problem of lack of practice.

Another problem intrinsic to the electronic sunglasses is that the liquid crystal panels have to be prepared and mounted on the frame separately for right and left eyes so that the transmittances of the right and left become different, if the axes of polarization are misaligned, to deteriorate the wearing feel seriously.

This problem can be solved if the right and left of polarization are aligned when the sunglasses are produced. This alignment could be completely effected only if the sunglasses were manufactured by those highly skilled in the art. However, the alignment cannot be attained by the mass production at a reasonable cost.

Thus, the electronic sunglasses using the liquid crystal panels have failed to be practiced by those various causes despite a number of engineers have proposed ideas and investigated for a long time.

## SUMMARY OF THE INVENTION

An object of the present invention is to solve those difficult problems and to provide electronic sunglasses which can satisfy the practical purposes.

According to a major aspect of the present invention, there is provided a pair of electronic sunglasses making use of the electrooptic effect of a liquid crystal, which comprise: a pair of liquid crystal panels of twist nematic type each made of a substrate of a synthetic resin for forming a transmittance-variable portion, said liquid crystal panels having a twist angle of an orientation of $60°$ to $80°$ or $100°$ to $120°$; a pair of polarizing plates applied to said liquid crystal panels and having their axes of absorption or polarization aligned with said angle of orientation of said liquid crystal panels, the smaller one of the angles formed between the axes of polarization of the objective polarizing plates of said liquid crystal panels for the right and left eyes being less than $45°$; and a solar cell acting as a power source for driving said liquid crystal panels.

According to another aspect of the present invention, the light receiving area S of said solar cell is specified by the following expression:
$S \leq 24$ cm$^2$.

The weight of spectacles, not limited to sunglasses, has an important meaning when they are actually worn. The heavier the spectacles, the worse the waering feel becomes, making it difficult to wear them for a long time. The weight desired for the glasses is 50 g or less, and the ordinary spectacles made of substrates of a synthetic resin have a weight of about 30 g. As different from the ordinary spectacles, the electronic sunglasses are desired to have their individual parts - liquid crystal panels, a power source unit, a drive-control circuit unit or their mounts - reduced as much as possible.

In the present invention, searches and investigations have been deeply made upon the method of reducing the weight of the electronic sunglasses for the liquid crystal panels and the solar cell, which are accepted as most effective components for the weight reduction.

The following results are obtained for the liquid crystal panels. Comparing the liquid crystal panels made of substrates of a synthetic resin and glass, it has been found that the glass substrates have a double weight for the common shape and thickness. The liquid crystal panels were trially manufactured and weighed. The lenses acting as the transmittance-variable portions having a thickness of 2 mm and an area of 26 cm$^2$ had weights of 16.2 g and 7.9 g for the glass and resin substrates, respectively. This means that the weight of the actual, electronic sunglasses could be reduced to about 16 g for the two eyes merely by changing the material of the liquid crystal panels from the glass to the synthetic resin.

Not only the weight but also the safety has to be considered because the liquid crystal panels are used as the transmittance-variable portions, i.e., lenses of the electronic sunglasses. If the substrates of the liquid crystal panels are made of glass, they are seriously dangerous, if broken. For safety, therefore, the transmittance-variable portions have to be made thicker than those of a synthetic resin. This increase in the weight causes a negative factor.

The liquid crystal panels of the resin can be produced by a variety of methods. In one of these, transparent electrode films such as ITO films are formed directly on the resin substrates and are then oriented. After this, the gap between the resin sub strates fixed by a spacer is filled up with a liquid crystal, and polarizing plates are applied to form the liquid crystal panels. Another method uses film-shaped liquid crystal panels which have their substrates made of films of a synthetic resin. Alternatively, substrates of a separately prepared synthetic resin are applied by a transparent adhesive to one or two sides of film-shaped liquid crystal panels, if these panels are worried about in the respect of strength, to provide the liquid crystal panels as the transmittance-variable portions. According to any of those methods, most of the transmittance-variable portions may be formed of the synthetic resin. The substrates may be shaped into flat or curved plates. In case the visual power is to be corrected, it is also possible to use lens-shaped substrates having a curvature conforming to the visible degree.

As to the solar cell, on the other hand, the weight can be reduced by regulating its light receiving or exposure area. The lower limit of the exposure area is determined by the performance of the solar cell and the electric power necessary for driving the liquid crystal panels, whereas the upper limit is restricted by the weight. In case the exposure area of the solar cell is to be specified, the weight balance with other components is determinant. Therefore, the weights of the components were examined, as follows.

The frame of the sunglasses is composed of rims supporting the transmittance-variable portions and bows to be put on the wearer's ears and has a weight of about 17 g no matter whether it is made of a resin or metal.

The weight of the transmittance-variable portions depends upon the area and thickness but mainly upon the thickness only for the ordinary sunglasses having a fixed area of about 26 cm². In case the liquid crystal panels are made of a synthetic resin, they should have a thickness of 2 mm at the least because they are liable to be deformed by an external force, as has been found by the investigations of applying various external forces to the liquid crystal panels. Thus, the weight of the liquid crystal panels having the transmittance-variable portions is about 8 g for one eye and about 16 g for two eyes.

This means that the total weight of the frame and the transmittance-variable portions is about 33 g. For providing sunglasses having an acceptable wearing feel, it is desirable that the total weight be 50 g or less. It is a condition that the total weight of the remaining components - the solar cell, the circuit and their mounts - be 50 g - 33 g = 17 g or less.

Since the weight of the circuit itself can be reduced to 1 g or less by the integration technique, it is necessary to suppress the weight of the mounts for the solar cell and the circuit within 16 g.

The mount for the solar cell has the larger area for the larger exposure area. Even in case the mount is made of a light, synthetic resin, the measurements have revealed that the mount and the solar cell have a substantially equal weight if the mount is required to have a practical strength. In other words, the electronic sunglasses can enjoy the acceptable wearing feel if the weight of the solar cell is 8 g or less.

The weight of the solar cell, which is prepared by forming amorphous silicon on the glass substrates so that it is inexpensive and is widely used, is about 0.3 g per cm² for a relatively large thickness of 1 to 1.2 mm. It follows that the exposure area of the solar cell be about 24 cm² or less.

This value of 24 cm² was determined as an average although the weights thus far specified will disperse depending upon the materials and sizes of the individual parts. The exceeding value would be undesired from the design standpoint.

In the present invention, moreover, the liquid crystal panels meeting the practical purposes as the electronic sunglasses have been prepared by improving the visual-angle dependency and the upper and lower limits of the variable transmittance of the liquid crystal panels of twist nematic type.

The major improvements are directed to the following two points:

1. The twist angle of the twist nematic system, and the included angle of the polarizing plates; and

2. The product $\Delta nd$ of the anisotropy $\Delta n$ of the refractive index and the distance d between the substrates sandwiching the liquid crystal.

In the twist nematic case, most of the twist angles and the included angles of the polarizing plates have been 90° for applications such as display elements. In the present invention, on the contrary, the twist angle of the liquid crystal panels of the twist nematic type has an orientation of 60° to 80° or 100° to 120, and the absorption or polarizing axes of the paired polarizing plates are aligned with that direction of orientation. In the sunglass case, the minimum transmittance is desirably 2 % at the least so as to prevent excessive darkness. The minimum transmittance for practical uses can be selected between 2 to 20 % by setting the twist angle and the included angle of the polarizing plate freely within the range of 60° to 80° or 100° to 120°. In order to reduce the visual-angle dependency thereby to improve the steepness of the voltage-transmittance characteristics, it is advisable to use the larger range of the twist angle within 100° to 120°.

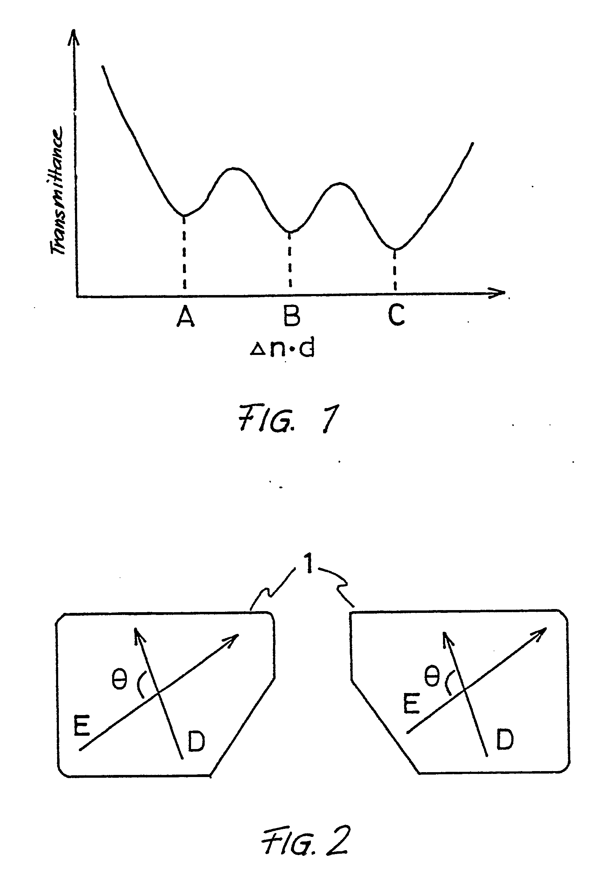

The product of Δnd is set at such a value as to reduce the visual-angle dependency. The value of Δnd is set at points A, B and C of Fig. 1, where the transmittance takes the minimal values on the Δnd dependency curve of the transmittance of the liquid crystal panels. The setting at the point A giving the minimum is not employed so much because the interelectrode distance of the liquid crystal panels is excessively short, and the setting at the points B and C is ordinarily employed because of feasible production. Despite of this fact, however, the transmittance at the point A giving the minimum of the value Δnd is desirably used in the present invention because the visual-angle dependency plays a major factor in the spectacles such as the electronic sunglasses. The value of Δnd may disperse about the point A by 0.25 times as high as the value at the point A..

Next, the present invention has considered the use of the polarizing plates in the spectacle lenses and makes investigations on the directions of the axes of polarizations of the polalizing plates to determine them for comfortable utilities as the spectacles.

The liquid crystal panels of the twist nematic type use two polarizing plates. If, at this instance, the objective polarizing plates for the right and left eyes have largely different directions in their polarizing axes, the transmittances to the reflected lights are accordingly different for the right and left eyes to give seriously uncomfortable feel if observations are actually made through the liquid crystal panels. When the reflected light was observed through the polarizing plates applied to the right and left eyes while being changed at the angles of their axes of polarization, the uncomfortable feel was minimized in case the smaller one of the angles included between the polarization axes of the right and left polarizing plates is less than 45°. The different feel was cleared at the angle of 20° or less, and it is desirable that the smaller one of the angles of the polarizing axes be 20° or less.

BRIEF DESCRIPTION OF THE DRAWINGS

Fig. 1 is a diagram plotting the relation between the transmittance of the liquid crystal panels and the product of Δnd;

Fig. 2 is a front elevation showing one embodiment of the liquid crystal panels according to the present invention;

Fig. 3 is a circuit diagram according to one embodiment of the electronic sunglasses according to the present invention;

Fig. 4 is a perspective view showing one embodiment of the electronic sunglasses according to the present invention;

Fig. 5 is a circuit diagram according to another embodiment of the electronic sunglasses according to the present invention; and

Fig. 6 is a perspective view showing another embodiment of the electronic sunglasses according to the present invention.

DESCRIPTION OF THE PREFERRED EMBODIMENTS

The present invention will be described in detail in the following in connection with the examples thereof, to which the present invention should not be limited.

Example-1

Fig. 2 is a front elevation showing the liquid crystal panels according to one embodiment of the present invention. The liquid crystal panels, as designated at numeral 1, were prepared, as follows. Between a substrate rubbed in a direction D and a substrate rubbed in a direction E, a nematic liquid crystal, to which a rightward rotatory "Kairal" substance having a refractive index anisotropy of Δn = 0.078 was added in a suitable amount, was scattered with an inter-substrate distance of 7 μ and sealed with a sealing agent such that the rubbing directions D and E made an angle θ of 110°. The substrates thus rubbed were made of films of a synthetic resin having electrode faces of ITO films. Next, the polarizing plates were adhered from the two sides such that their polarization axes were aligned with the aforementioned rubbing directions. The polarizing plate to be applied to the substrate rubbed in the direction D has its polarization axis aligned with the direction D, whereas the polarizing plate to be applied to the substrate rubbed in the direction E has its polarization axis aligned with the direction E. As seen from Fig. 2, the directions of the polarization axes for the right and left eyes are aligned with each other, i.e. in this case the smaller one of the angles formed between the axes of polarization of the objective polarizing plates of said liquid crystal panels for the right and left eyes is zero, which is the most preferable case. Since the liquid crystal panels thus prepared are fragile and liable to be damaged when used as the transmittance-variable portions of the sunglasses, acrylic plates having a thickness of 1 mm were applied to the two sides of the panels by means of an adhesive of ultraviolet setting type. The remaining outer sides were hardened in advance with hard coatings of ultraviolet setting type.

The transmittance-variable portions thus prepared had a transmittance of 8.11 %, when supplied with a voltage of 5 V (static), and a transmittance of 35 % when released. The driving characteristics obtained were such that the transmittance was saturated at 8 % for a current consumption of 50 $\mu$A when supplied with a voltage of 2.5 to 3 V. According to these driving characteristics, the circuit was designed to determine the light receiving or exposure area of the solar cell.

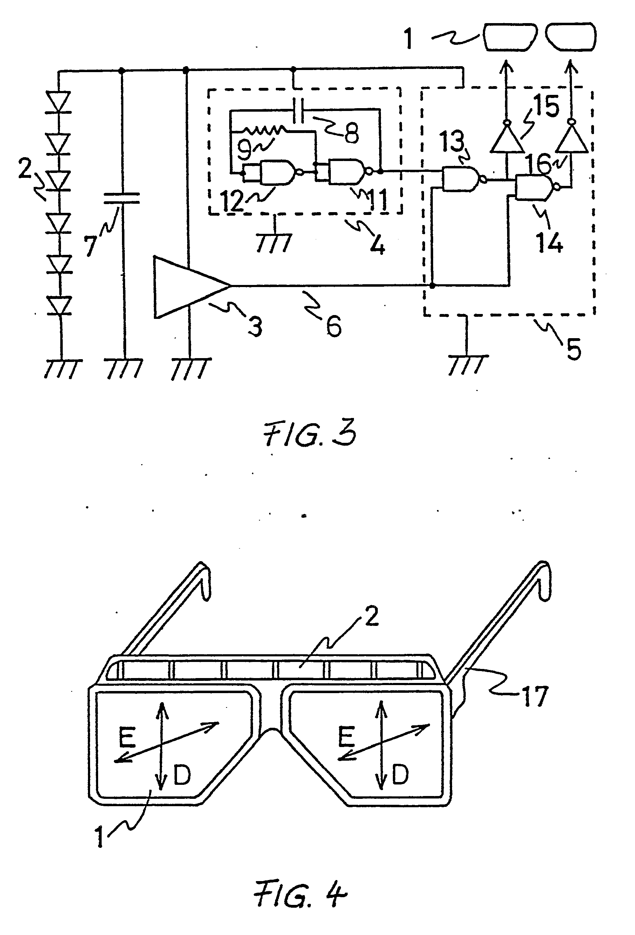

Fig. 3 presents a circuit diagram showing one embodiment of the present invention. This circuit is composed of a solar cell 2, a voltage detector 3, an oscillating circuit 4, a liquid crystal driving circuit 5, and the liquid crystal panels 1.

The oscillating circuit 4 is a CR oscillator composed of NAND gates 11 and 12, a capacitor 8 and a resistor 9. (The oscillating circuit should not be limited by the present invention but is presented merely for illustrations.) On the other hand, the liquid crystal driving circuit 5 is composed of NAND gates 13 and 14 for controlling the output and drivers 15 and 16. The details of the voltage detector 3 will be omitted. A power source protecting capacitor 7 is connected in parallel with the circuit.

The operations will be described in the following with reference to Fig. 3. The solar cell 2 used was made of amorphous silicon and had an electromotive force of 0.7 V and a current value of 700 $\mu$A for an irradiation of 10,000 Luxes per cm². The output voltage of 3 V will be sufficient for driving the liquid crystal panels but was so set as to produce an output voltage of 4 V or more for the illuminance of 10,000 Luxes. Therefore, the solar cell used had an exposure area of 6 cm² by connecting six cells having an area of 1 cm² in series. If the critical level of the voltage detector is set at 4 V, an output signal 6 in this state is off so that the liquid crystal panels 1 are supplied with a voltage in phase and not lit. If the illuminance exceeds 10,000 Luxes, the electromotive force of the solar cell exceeds 4 V so that the output signal 6 of the voltage detector 3 is turned on to apply the AC signal of the oscillating circuit 4 in the opposite phase from the inverters 15 and 16 to the liquid crystal cells. The driving frequency is suitably determined at several ten to several hundreds Hz by the resistor 9 and the capacitor 8. The power source protecting capacitor 7 eliminates the noises or ripples to the power source and has a suitable capacity of several tens $\mu$F. The voltage detector 3 is well known as various power source ICs or power source voltage detectors, and its detailed description will be omitted.

As an application of the present invention, moreover, the voltage detector can be so improved by providing means to allow that the user himself can adjust the detection level. It is also possible to give a hysteresis to the detection level so that the user's eyes may grow familiar to an optical change. For example, the illuminance for starting the screening is set at a high level (e.g., a high voltage for ON) when he comes out from a dark to a bright place. In the reverse case, the illuminance is set at a low level (e.g., a low voltage for OFF). This setting provides counter-measures for preventing the user from being dazzled under critical conditions.

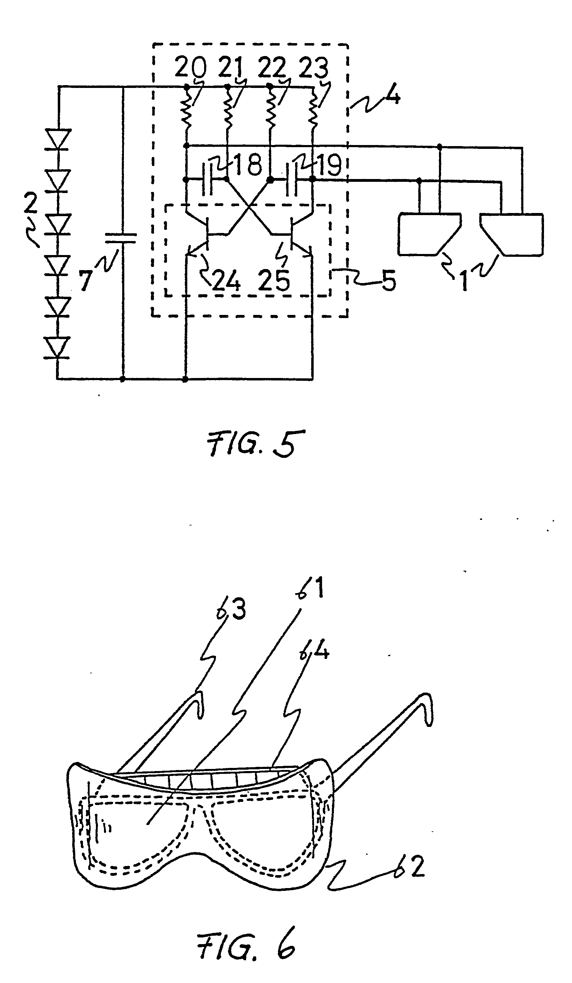

Fig. 4 is a perspective view showing one embodiment of the present invention, in which the solar cell 2, the circuit and the liquid crystal panels 1 are built in a frame 17. Arrow D designates the axis of polarization of the objective side polarizing plate, and arrow E designates the axis of polarization of the the eye side polarizing plate. The polarization axes of the objective side polarizing plates for the right and left eyes were arranged in the same vertical direction. Moreover, the flat planes of the right and left transmittance-variable portions were arranged not in a common plane but to have their normals make an angle of 25°. In case the transmittance-variable portions were to be built in the frame, an elastic adhesive was used to free the portions from any stress from the frame. The total weight of the assembly was 35.2 g and is small for the spectacles to give a good wearing feel.

Example-2

The flat, acrylic plates of 1 mm of the Example 1, which were adhered to the film-shaped liquid crystal panels, were replaced by lenses of diethylene glycol bisalyl carbonate resin to provide dioptric transmittance-variable portions. These lenses were prepared by thermal polymerizations. As a matter of fact, the following lenses were adhered to the objective and eye sides of the liquid crystal panels to make them diopric as a whole. The lens to be adhered to the objective side had a radius of convex curvature of 13 cm on one side, an infinite (i.e. flat) radius of curvature on the other side, and a center thickness of 5 mm, whereas the lens to be adhered to the eye side had an infinite radius of curvature on one side, a radius of concave curvature of 10 cm on the other side, and a center thickness of 1 mm. The refractive power of the transmittance-variable portions thus prepared had a value of -1.14 diopters.

The transmittance-variable portions obtained were built in the frame together with the solar cell and the circuit, like the Example 1, to produce the sunglasses.

Example-3

The electrode substrates themselves of the liquid crystal panels were shaped into lenses to make the transmittance-variable portions dioptric. One substrate of the liquid crystal panels was prepared by forming a mold having a radius of convex curvature of 5 cm and a radius of concave curvature of 7.5 cm, by fixing a polarizing plate in the mold, and by injecting and thermally polymerizing a diethylene glycol bisalyl carbonate resin and a small amount of additive. The other substrate was prepared by forming a mold having a radius of convex curvature of 7.5 cm and a radius of concave curvature of 10 cm and by burying and thermally polymerizing a polarizing plate like the foregoing substrate. Next, an ITO film was formed over the face having the radius of curvature of 7.5 cm by a cold sputtering and was oriented. The rubbing direction for the orientation was aligned with the absorption or polarizing axes of the polarizing plates of the substrates. After this, glass beads having diameter of 7 μ were scattered all over the gap, while the faces having the radius of curvature of 7.5 cm opposing each other, to fix the twist angle at 70°. A TN type liquid crystal was adsorbed in a vacuum atmosphere by prepared pin holes to close these holes and was annealed at 60°C to form the transmittance-variable portions. The lenses had a refractive power of +5.1 diopters.

The transmittance-variable portions thus prepared were built in the frame together with the solar cell and the circuit like the Example 1 to complete the sunglasses.

Example-4

The electronic sunglasses were prepared absolutely like the Example 1 excepting the replacement of the circuit structure used in the Example 1 by that shown in Fig. 5. This circuit structure is constructed of: the solar cell 2; the liquid crystal panels 1; a power source stabilizing capacitor 7; the oscillating circuit 4 composed of capacitors 18 and 19, resistors 20, 21, 22 and 12 and transistors 24 and 25; and the liquid crystal driving circuit 5 composed of the transistors 24 and 25. When the solar cell 2 is exposed to no light, any voltage is not generated so that the oscillating circuit composed of the capacitors 18 and 19, the resistors 20, 21, 22 and 23 and the transistors 24 and 25 does not oscillate. As a result, the transmittance-variable portions are left in a high-transmittance state. If the solar cell 2 is exposed to a light of 1,000 to 5,000 Luxes, the transistors 24 and 25 oscillate, but the crest value of the collector voltage does not reach the ON potential so that the transmittance does not change. If the solar cell 2 is exposed to a light of about 6,000 Luxes or more, the crest value reaches the ON voltage so that the transmittance begins its change. With a more intense light, the transmittance-variable portions grow denser but are saturated for a light of 10,000 Luxes or more. Unlike the Example 1, the transmittance of the transmittance-variable portions is continuously changed depending upon the intensity of the ambient light.

Example-5

The electronic sunglasses were constructed like the Example 1 except that the following reflection preventing films were formed at the eye sides of the half hoods of hard-coated acrylic plates adhered to the two sides so as to fix the film-shaped liquid crystal panels of Example 1.

Prior to the adhesion to the liquid crystal panels, there were applied over the hard coating by a vacuum evaporation: a layer of $SiO_2$ having a thickness of $\lambda/4$ (wherein $\lambda$ = 520 nm); layers of $ZrO_2$ and $SiO_2$ having a total thickness of $\lambda/4$; a layer of $ZrO_2$ having a thickness of $\lambda/4$; and an uppermost layer of $SiO_2$ of $\lambda/4$ which are recited from the side of the synthetic resin substrates. In case the lenses themselves are colored like the sunglasses, their optical absorptivity is so that the reflections at the eye sides are prominent. In the case of the electronic sunglasses, too, less reflections from the backs will enter the eyes to eliminate the uncomfortableness merely by forming reflection preventing films on the eye sides.

Example-6

The film-shaped liquid crystal panels prepared in the Example 1 were sandwiched between two cylindrical diethylene glycol bisalyl carbonate plates having a radius of curvature of about 100 mm and a thickness of 1 mm and were adhered by means of an ultraviolet setting type adhesive. The transmittance-variable portions thus prepared were arranged to have the cylindrical curve directed vertically and were built in the frame. The axes of the polarization of the polarizing plates used were similar to those of the Example 1, and the other arrangements were also similar to those of the Example 1.

Example-7

The electronic sunglasses, which used the transmittance-variable portions having the flat acrylic plates adhered to the film-shaped liquid crystal panels as in the Example 1, are advantageous in that they could be produced more easily than the curved sunglasses of the Example 6 to provide more inexpensive sunglasses. However, the sunglasses having the flat lenses are short of the thickness required thereof to lose the high quality. Moreover, the restrictions on the design are too serious to provide excellently fashionable sunglasses.

In the present embodiment, therefore, a suitably curved polycarbonate sheet 62 was attached to the front side of flat transmittance-variable portions 61, as shown in Fig. 6. Numeral 63 designates a frame, and numeral 64 designates a solar cell. Although not shown, the circuit unit is built in like the Example 1.

The polycarbonate sheet 62 may be suitably shaped according to the designing requirement and may have a free size. The material should not be limited to the polycarbonate but may be suitably selected from a light, synthetic resin such as acryl. The material may be suitably colored, as required. The attachment should not be especially limited but may be exemplified by the adhering, caulking or screwing. If the sheet is made removable, it can be replaced by another in accordance with the application so that the sunglasses can be better fashionable.

According to the present invention, the weight of the electronic sunglasses can be reduced to 50 g or less by making the substrates of a synthetic resin to prepare the liquid crystal panels constituting the transmittance-variable portions thereby to regulate the exposure area of the solar cell. As a result, the sunglasses can be worn for a long-time practical use without any fatigue. Since, moreover, the transmittance-variable portions are made of the synthetic resin, their strength against breakage is increased over the case of glass so that the safety is improved.

In the twist nematic type, moreover, the twist angle is oriented in a direction of 60° to 80° or 100° to 120°, and the absorption or polarization axes of the paired polarizing plates are aligned with the orienting direction so that the minimum transmittance can be adjusted while allowing the transmittance varying range to be freely set. As a result, it is possible to provide the transmittance-variable portions which can be actually used in the transmittance-variable, electronic sunglasses. If, moreover, the value of $\Delta$nd is set at that of the point A of Fig. 1, at which the transmittance takes the minimum on the $\Delta$nd dependency curve of the transmittance of the liquid crystal panels, i.e., at which the corresponding the $\Delta$nd value takes the minimum, and if the twist angle and the included angle of the polarizing plates are set within the ranges specified hereinbefore, the visual angle dependency of the liquid crystal panels can be improved. As a result, the unevenness and shading resulting from the change in the angle viewing the liquid crystal panels as the eyes turn can be eliminated to provide the electronic sunglasses which have no uncomfortable feel when worn.

Since, moreover, the smaller one of the angles made between the polarizing axes of the objective side polarizing plates of the liquid crystal panels for the right and left eyes is set at 45° or less, the difference between the quantities of the reflected incident lights is eliminated between the right and left eyes so that the uncomfortable feel for the external view to look stereoscopic can be eliminated to provide practically acceptable, electronic sunglasses.

As having been described hereinbefore, according to the present invention, there is provided electronic sunglasses which can be worn for a long time without the uncomfortable feel.

**Claims**

1. A pair of electronic sunglasses making use of the electrooptic effect of a liquid crystal, comprising:
a pair of liquid crystal panels of twist nematic type each made of a substrate of a synthetic resin for forming a transmittance-variable portion, said liquid crystal panels having a twist angle of an orientation of 60° to 80° or 100° to 120°;
a pair of polarizing plates applied to said liquid crystal panels and having their axes of absorption or polarization aligned with said angle of orientation of said liquid crystal panels, the smaller one of the angles formed between the axes of polarization of the objective polarizing plates of said liquid crystal panels for the right and left eyes being less than 45°; and
a solar cell acting as a power source for driving said liquid crystal panels.

2. A pair of electronic sunglasses according to Claim 1, wherein the light receiving area S of said solar cell is specified by the following expression: $S \leq 24$ cm$^2$.

FIG. 1

FIG. 2

FIG. 3

FIG. 4

FIG. 5

FIG. 6